# EUROPEAN PATENT APPLICATION

(11) **EP 4 674 838 A1**
(43) Date of publication of application: **07.01.2026**
(21) Application number: 24763764.8
(22) Date of filing: 22.02.2024
(51) Int. Cl.: C07D 201/12

(54) **METHOD FOR PRODUCING CYCLIC LACTAM AND APPARATUS FOR PRODUCING CYCLIC LACTAM**

(30) Priority: 27.02.2023 JP 2023028312
(71) Applicant: MITSUBISHI GAS CHEMICAL COMPANY, INC., Chiyoda-ku Tokyo 100-8324 (JP)
(72) Inventor: AOYAGI, Naoto, Tokyo 125-8601 (JP); TOKUDA, Yuka, Tokyo 125-8601 (JP); HIRAYAMA, Arinobu, Yokkaichi-shi, Mie 510-0886 (JP)
(74) Representative: Vossius & Partner Patentanwälte Rechtsanwälte mbB
(86) International application number: PCT/JP2024/006441
(87) International publication number: WO 2024/181283

(57) **Abstract**

To provide a method for manufacturing a cyclic lactam that can manufacture the cyclic lactam with high selectivity, and a device for manufacturing a cyclic lactam. A method for manufacturing a cyclic lactam, the method including performing a depolymerization reaction by using 1 part by mass or more of a polyphosphoric acid per 100 parts by mass of a polyamide resin containing a repeating unit represented by Formula (a). In Formula (a), n1 is an integer of 3 to 22, and n1 may be a different value for each of the repeating unit.

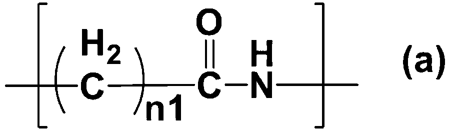

## Description

### Technical Field

The present invention relates to a method for manufacturing a cyclic lactam and a device for manufacturing a cyclic lactam. The present invention particularly relates to a method for manufacturing a cyclic lactam by a depolymerization reaction of a polyamide resin, and the like.

### Background Art

Depolymerization of a polyamide resin to recover a cyclic lactam has been studied.

For example, Patent Document 1 describes a method of recovering ε-caprolactone and ε-caprolactam from a mixture containing ε-oxycaproamide and an oligomer of ε-caprolactam. The method is characterized by bringing the mixture into contact with water vapor at a temperature of 200 to 360°C in the presence of phosphoric acids.

Furthermore, Patent Document 2 describes a method for purifying ε-caprolactam. The method is characterized by depolymerizing a thermoplastic material containing nylon 6 as a main component, recovering ε-caprolactam, and rectifying the recovered ε-caprolactam.

### Citation List

### Patent Document

Patent Document 1: JP S49-132094 A
Patent Document 2: JP H08-217746 A

### Summary of Invention

### Technical Problem

Note that Patent Document 1 describes that, at the time of depolymerization of the polyamide resin, phosphoric acids, such as orthophosphoric acid, orthophosphoric acid, pyrophosphoric acid, and ammonium phosphate, are used as catalysts. Furthermore, Patent Document 2 also describes depolymerization of the polyamide resin by using phosphoric acid. However, when the inventors of the present invention conducted a study, it was found that the yield of caprolactam, which is a product, is low when such phosphoric acids are used.

The present invention is to solve the issue described above, and an object of the present invention is to provide a method for manufacturing a cyclic lactam that can manufacture the cyclic lactam with high selectivity, and a device for manufacturing a cyclic lactam.

### Solution to Problem

As a result of studies conducted in response to the issue described above, the present inventors found that the above issue can be solved by performing a depolymerization reaction of the polyamide resin by using a polyphosphoric acid.

Specifically, the aforementioned issue is solved by the following means.
<1> A method for manufacturing a cyclic lactam, the method including performing a depolymerization reaction by using 1 part by mass or more of a polyphosphoric acid per 100 parts by mass of a polyamide resin containing a repeating unit represented by Formula (a): 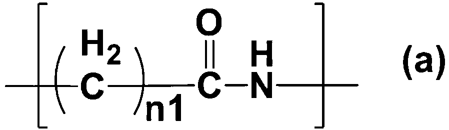 where n1 is an integer of 3 to 22, and n1 may be a different value for each of the repeating unit.
<2> The method for manufacturing a cyclic lactam according to <1>, wherein 40 parts by mass or more of the polyphosphoric acid is used per 100 parts by mass of the polyamide resin.
<3> The method for manufacturing a cyclic lactam according to <1> or <2>, wherein from 50 to 10000 parts by mass of the polyphosphoric acid is used per 100 parts by mass of the polyamide resin.
<4> The method for manufacturing a cyclic lactam according to any one of <1> to <3>, wherein the depolymerization reaction is performed under a pressure of 202.6 kPa or less.
<5> The method for manufacturing a cyclic lactam according to any one of <1> to <4>, wherein reactive distillation is performed while the depolymerization reaction is performed.
<6> The method for manufacturing a cyclic lactam according to any one of <1> to <5>, wherein distillation is performed after the depolymerization reaction is performed.
<7> The method for manufacturing a cyclic lactam according to any one of <1> to <6>, wherein the depolymerization reaction is performed at a temperature of 170°C to 300°C.
<8> The method for manufacturing a cyclic lactam according to any one of <1> to <7>, wherein superheated steam is not used when the depolymerization reaction is performed.
<9> The method for manufacturing a cyclic lactam according to any one of <1> to <8>, wherein, when the depolymerization reaction is performed, no solvent is used or an amount of a solvent is 10 mass% or less with respect to an amount of the polyphosphoric acid.
<10> The method for manufacturing a cyclic lactam according to any one of <1> to <9>, wherein the depolymerization reaction is performed in the presence of a solvent having a high boiling point, and a boiling point of the solvent having the high boiling point is not lower than a temperature that is 10°C lower than a boiling point of a cyclic lactam to be manufactured.
<11> The method for manufacturing a cyclic lactam according to <10>, wherein the solvent having the high boiling point is not an alcohol.
<12> The method for manufacturing a cyclic lactam according to any one of <1> to <11>, wherein
   reactive distillation is performed while the depolymerization reaction is performed;
   the depolymerization reaction is performed at a temperature of 170°C to 300°C;
   when the depolymerization reaction is performed, superheated steam is not used; and
   when the depolymerization reaction is performed, no solvent is used or an amount of a solvent is 10 mass% or less with respect to an amount of the polyphosphoric acid.
<13> The method for manufacturing a cyclic lactam according to any one of <1> to <11>, wherein
   reactive distillation is performed while the depolymerization reaction is performed;
   the depolymerization reaction is performed at a temperature of 170°C to 300°C;
   when the depolymerization reaction is performed, superheated steam is not used;
   the depolymerization reaction is performed in the presence of a solvent having a high boiling point, and a boiling point of the solvent having the high boiling point is not lower than a temperature that is 10°C lower than a boiling point of a cyclic lactam to be manufactured; and
   the solvent having the high boiling point is not an alcohol.
<14> The method for manufacturing a cyclic lactam according to any one of <1> to <11>, wherein,
   after the depolymerization reaction is performed, distillation is performed;
   the depolymerization reaction is performed at a temperature of 170°C to 300°C;
   when the depolymerization reaction is performed, superheated steam is not used; and
   when the depolymerization reaction is performed, no solvent is used or an amount of a solvent is 10 mass% or less with respect to an amount of the polyphosphoric acid.
<15> The method for manufacturing a cyclic lactam according to any one of <1> to <11>, wherein,
   after the depolymerization reaction is performed, distillation is performed;
   the depolymerization reaction is performed at a temperature of 170°C to 300°C;
   when the depolymerization reaction is performed, superheated steam is not used;
   the depolymerization reaction is performed in the presence of a solvent having a high boiling point, and a boiling point of the solvent having the high boiling point is not lower than a temperature that is 10°C lower than a boiling point of a cyclic lactam to be manufactured; and
   the solvent having the high boiling point is not an alcohol.
<16> The method for manufacturing a cyclic lactam according to any one of <1> to <15>, wherein a molecular weight of the polyphosphoric acid is 177 or more and 10000 or less.
<17> The method for manufacturing a cyclic lactam according to any one of <1> to <15>, wherein the polyamide resin is at least one type selected from the group consisting of nylon 4, nylon 5, nylon 6, nylon 11, and nylon 12.
<18> The method for manufacturing a cyclic lactam according to any one of <1> to <17>, wherein a cyclic lactam is manufactured by continuously supplying the polyamide resin to a reaction system.
<19> The method for manufacturing a cyclic lactam according to any one of <1> to <18>, wherein the polyphosphoric acid is directly supplied to a reaction system.
<20> The method for manufacturing a cyclic lactam according to <19>, wherein a water content of the polyphosphoric acid supplied to the reaction system is 100 mass% or less with respect to a mass of the polyphosphoric acid.
<21> The method for manufacturing a cyclic lactam according to <19> or <20>, wherein a polyphosphoric acid (B2) having a degree of polymerization higher than a degree of polymerization of the polyphosphoric acid supplied to the reaction system is hydrolyzed and a polyphosphoric acid (B3) is regenerated.
<22> The method for manufacturing a cyclic lactam according to <19> or <20>, wherein a polyphosphoric acid (B2) having a degree of polymerization higher than a degree of polymerization of the polyphosphoric acid supplied to the reaction system is hydrolyzed and a regenerated polyphosphoric acid (B3) is regenerated in the reaction system.
<23> The method for manufacturing a cyclic lactam according to <21>, wherein the polyphosphoric acid (B3) is used as a polyphosphoric acid to be supplied to the reaction system.
<24> A device for manufacturing the cyclic lactam configured to perform the method for manufacturing a cyclic lactam described in any one of <1> to <23>, the device including:
   (1) a reactor configured to perform a depolymerization reaction by heating a mixture A containing a polyamide resin containing a repeating unit represented by Formula (a) and a polyphosphoric acid, to produce a cyclic lactam, and to form a mixture B containing the polyphosphoric acid and the cyclic lactam;
   (2) a container configured to hold the cyclic lactam vaporized from the mixture B in the reactor; and
   (3) a flow pass configured to connect the reactor and the container and to transfer the vaporized cyclic lactam from the reactor to the container.
<25> The device for manufacturing a cyclic lactam according to <24>, further including a pressure regulator configured to maintain a pressure of 202.6 kPa or less in the reactor, the container, and the flow pass.
<26> The device for manufacturing a cyclic lactam according to <25>, wherein the pressure regulator is connected to at least one selected from the group consisting of the reactor, the container, and the flow pass.
<27> The device for manufacturing a cyclic lactam according to any one of <24> to <26>, further including a heated part configured to maintain a temperature in the flow pass to not lower than a melting point of the cyclic lactam.
<28> The device for manufacturing a cyclic lactam according to any one of <24> to <26>, further including an apparatus for removing water from the reactor.

### Advantageous Effects of Invention

The present invention can provide a method for manufacturing a cyclic lactam that can manufacture the cyclic lactam with high selectivity, and a device for manufacturing a cyclic lactam.

### Brief Description of Drawings

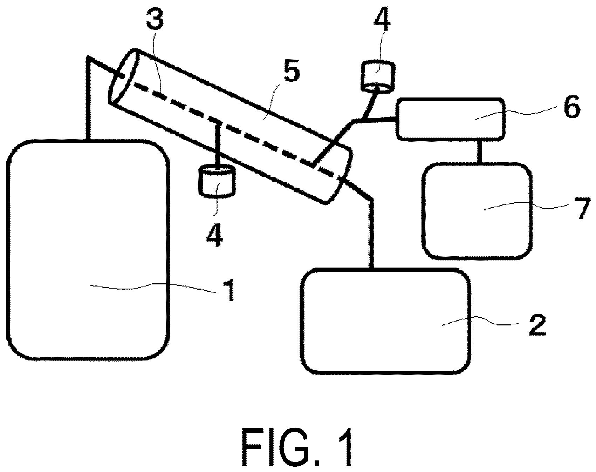

FIG. 1 illustrates a schematic view of an example of a device for manufacturing a cyclic lactam of the present embodiment.

### Description of Embodiments

Embodiments for carrying out the present invention (hereinafter, referred to simply as "the present embodiment") will next be described in detail. The present embodiment described below is an example for describing the present invention, and the present invention is not limited only to the present embodiment.

In the present specification, numerical values described before and after the term "to" are respectively the lower limit and the upper limit of a range including the numerical values.

In the present specification, the number average molecular weight is a value measured by the following method unless otherwise particularly noted.

The number average molecular weight (Mn) of the polyphosphoric acid is determined using an ion chromatography method in which a component constituting the polyphosphoric acid is separated and quantified. Measurement is performed by an electric conductivity detector using an anion-exchange packed column as a column, using an aqueous potassium hydroxide solution as an eluent, with a sample injection amount of 100 µL, at a column temperature of 35°C, and an eluent flow rate of 1.0 mL/min. The abundance ratio of each constituent is determined by calculating a proportion of an area of each peak taking a total area of all peaks as 100%.

In the present invention, the boiling point means a boiling point at a pressure of 101.33 kPa unless otherwise particularly noted.

The term "step" as used herein includes not only an independent step but also a step that cannot be clearly distinguished from other steps as long as an intended action of the step is achieved.

When the measurement methods or the like described in the standards found in the present description differ depending on the year, the measurement methods or the like are based on the standards as of January 1, 2023, unless otherwise particularly noted.

FIG. 1 is a schematic diagram, and scales and the like may be not consistent with reality.

The method for manufacturing a cyclic lactam of the present embodiment is characterized by performing a depolymerization reaction by using 1 part by mass or more of a polyphosphoric acid per 100 parts by mass of a polyamide resin containing a repeating unit represented by Formula (a) (hereinafter, may be referred to as "polyamide resin (a)").

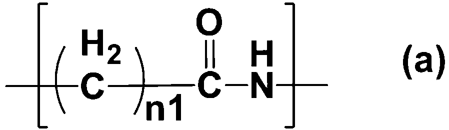

In Formula (a), n1 is an integer of 3 to 22, and n1 may be a different value for each of the repeating unit.

By such a configuration, a cyclic lactam can be manufactured with high selectivity.

In the method for manufacturing a cyclic lactam of the present embodiment, typically, the polyphosphoric acid is in a liquid state, and a depolymerization reaction progresses in a state where the polyamide resin (a) is dissolved. That is, it is presumed that hydrolysis using water does not occur but a backbiting reaction, in which an amino group at a terminal or an amide bond in a main chain of the polyamide resin (a) attacks an amide bond in one unit next to itself, proceeds. Furthermore, because the polyphosphoric acid has a mild acidity, the bonding strength to an amino group is weaker than a typical acid, and a cyclic amide structure tends to be formed during depolymerization reaction. That is, it is presumed that, because the polyphosphoric acid functions as a solvent and an acid catalyst, a cyclic lactam can be manufactured with high selectivity by performing a depolymerization reaction by using 1 part by mass or more of a polyphosphoric acid per 100 parts by mass of a polyamide resin containing a repeating unit represented by Formula (a).

In particular, because the polyphosphoric acid can function also as a solvent, the reaction efficiently proceeds without use of a solvent having a boiling point lower than that of the cyclic lactam, such as water or an alcohol, and the formed cyclic lactam can be purified by a simple and easy step, such as simple distillation.

Hereinafter, the present invention will be described in detail.

### Polyamide Resin Containing Repeating Unit Represented by Formula (a) (Polyamide Resin (a))

In the present embodiment, the polyamide resin (a) used in the depolymerization reaction contains a repeating unit represented by Formula (a).

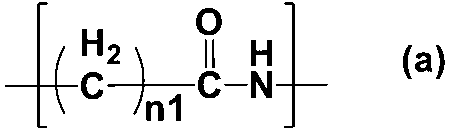

In Formula (a), n1 is an integer of 3 to 22, and n1 may be a different value for each of the repeating unit.

n1 is preferably 4 or more, and more preferably 5 or more, and preferably 20 or less, more preferably 18 or less, even more preferably 16 or less, yet even more preferably 14 or less, yet even more preferably 12 or less, yet even more preferably 10 or less, yet even more preferably 8 or less, and particularly preferably 6 or less.

The proportion of the repeating unit represented by Formula (a) in the polyamide resin (a) is preferably 80 mass% or more, more preferably 90 mass% or more, even more preferably 95 mass% or more, and yet even more preferably 99 mass% or more, of all repeating units of the polyamide resin (a).

Furthermore, terminals of the polyamide resin (a) may be -NH₂ and/or -COOH or may be capped with a terminal blocking agent.

The polyamide resin (a) is preferably at least one type selected from the group consisting of nylon 4, nylon 5, nylon 6, nylon 11, and nylon 12, and more preferably nylon 6.

Note that the polyamide resin (a) may be synthesized from a cyclic lactam or may be synthesized from an aminocarboxylic acid, and is preferably synthesized from a cyclic lactam. Furthermore, the nylon 6 in the present embodiment includes those having a part (e.g., 5 mass% or less, 3 mass% or less, and especially 1 mass% or less) modified with another monomer. The same applies to the other nylons.

In the method for manufacturing a cyclic lactam of the present embodiment, one type of the polyamide resin (a) may be used, or two or more types of the polyamide resins (a) may be used.

The viscosity average molecular weight of the polyamide resin (a) is preferably 3000 or more, more preferably 5000 or more, and even more preferably 10000 or more, and may be 50000 or more, and is preferably 1000000 or less, more preferably 500000 or less, and even more preferably 100000 or less.

The viscosity average molecular weight is measured in accordance with JIS K 7367.

In the method for manufacturing a cyclic lactam of the present embodiment, a polyamide resin containing no repeating unit represented by Formula (a) (additional polyamide resin) may be contained in addition to the polyamide resin containing the repeating unit represented by Formula (a) in a range not departing from the scope of the present invention. Examples of the additional polyamide resin include nylon 66, nylon 666, and semiaromatic polyamide resins. However, in the method for manufacturing a cyclic lactam of the present embodiment, an amount of the additional polyamide resin supplied to the reaction system is preferably 5 mass% or less, more preferably 3 mass% or less, and even more preferably 1 mass% or less, of the amount of the polyamide resin (a).

Furthermore, in the method for manufacturing a cyclic lactam of the present embodiment, an additional thermoplastic resin other than the polyamide resin may be contained in addition to the polyamide resin (a) in a range not departing from the scope of the present invention. Examples of such an additional thermoplastic resin include a polypropylene resin, a polyolefin resin such as a polyethylene resin, and a polyester resin such as a polyethylene terephthalate resin. In the method for manufacturing a cyclic lactam of the present embodiment, an amount of such an additional thermoplastic resin supplied to the reaction system may be, for example, 5 mass% or less, less than 5 mass%, 3 mass% or less, or especially 1 mass% or less, of the amount of the polyamide resin (a).

### Polyphosphoric Acid

In the method for manufacturing a cyclic lactam of the present embodiment, a depolymerization reaction is performed by using 1 part by mass or more of a polyphosphoric acid per 100 parts by mass of the polyamide resin containing the repeating unit represented by Formula (a).

The polyphosphoric acid acts as a catalyst in a ring-opening reaction of the amide bond in the polyamide resin (a) and dissolves the polyamide resin (a), and thus the polyphosphoric acid contributes to decrease in the viscosity of the reaction liquid. Thus, stirring of the reaction liquid is facilitated, and a cyclic lactam can be efficiently produced. The polyphosphoric acid is preferably directly supplied to the reaction system (e.g., reaction tank).

The polyphosphoric acid may be a linear polyphosphoric acid and a cyclic polyphosphoric acid, and is preferably a linear polyphosphoric acid.

Specifically, the polyphosphoric acid is preferably at least one type selected from the group consisting of pyrophosphoric acid, tripolyphosphoric acid, a medium-long chain polyphosphoric acid having from 4 to 100 phosphoric acid moieties, and trimetaphosphate (six-membered ring), and more preferably at least one type selected from the group consisting of pyrophosphoric acid, tripolyphosphoric acid, and a medium-long chain polyphosphoric acid having from 4 to 100 phosphoric acid moieties.

More specifically, the polyphosphoric acid preferably includes a compound represented by Formula (P) below.

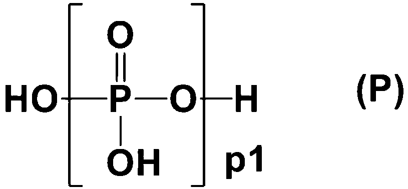

In Formula (P), p1 is preferably an integer of 2 to 100.

In the compound represented by Formula (P), p1 is preferably 3 or more, and more preferably 4 or more, and may be 5 or more. When p1 is not lower than the lower limit, promotion of the depolymerization reaction by high catalytic activity and selectivity of the cyclic lactam tend to further improve. In the compound represented by Formula (P), p1 is preferably 90 or less, more preferably 70 or less, even more preferably 50 or less, yet even more preferably 30 or less, yet even more preferably 20 or less, and yet even more preferably 10 or less. When p1 is not higher than the upper limit, the viscosity of the reaction liquid becomes more appropriate, and efficiency of dissolution of the polyamide resin (a) and the depolymerization reaction tend to further improve.

The polyphosphoric acid may be made of one type of compound represented by Formula (P) or may be a mixture of two or more types of compounds represented by Formula (P). Furthermore, the polyphosphoric acid may contain a compound in which p1 in Formula (P) is 1, or may contain a compound in which p1 in Formula (P) is greater than 100.

In a case where the polyphosphoric acid includes a compound represented by Formula (P) above but does not include a compound in which p1 in Formula (P) is 1 or more and/or a compound in which p1 in Formula (P) is greater than 100 and, further, in a case where the polyphosphoric acid does not include both of a compound in which p1 in Formula (P) is 1 or more and a compound in which p1 in Formula (P) is greater than 100, an average value of p1 in Formula (P) is preferably in a range of p1 of the compound represented by Formula (P) described above.

Furthermore, in a case where the polyphosphoric acid includes a compound represented by Formula (P) above and includes a compound in which p1 in Formula (P) is 1 or more and/or a compound in which p1 in Formula (P) is greater than 100 and, further, in a case where the polyphosphoric acid includes both of a compound in which p1 in Formula (P) is 1 or more and a compound in which p1 in Formula (P) is greater than 100, an average value of p1 in Formula (P) is preferably 1.5 or more, more preferably 2.0 or more, even more preferably 2.5 or more, and yet even more preferably 3.0 or more, and may be 5.0 or more, and is preferably 100 or less, more preferably 70 or less, even more preferably 50 or less, yet even more preferably 30 or less, yet even more preferably 20 or less, and yet even more preferably 10 or less.

The molecular weight of the compound represented by Formula (P) is preferably 177 or more, and more preferably 257 or more. When the molecular weight is not lower than the lower limit, the depolymerization reaction is more effectively promoted due to high catalytic activity, and thus a cyclic lactam tends to be formed with higher selectivity. Furthermore, the molecular weight of the compound represented by Formula (P) is preferably 10000 or less, more preferably 7000 or less, even more preferably 5000 or less, yet even more preferably 3000 or less, and yet even more preferably 1000 or less. When the molecular weight is not higher than the upper limit, the viscosity of the reaction liquid becomes more appropriate, and efficiency of dissolution of the polyamide resin (a) and the depolymerization reaction tend to further improve.

The number average molecular weight of the polyphosphoric acid is preferably 137 or more, and more preferably 177 or more. When the number average molecular weight is not lower than the lower limit, the depolymerization reaction is more effectively promoted due to high catalytic activity, and thus a cyclic lactam tends to be formed with higher selectivity. Furthermore, the number average molecular weight of the polyphosphoric acid is preferably 8017 or less, more preferably 5617 or less, even more preferably 4018 or less, yet even more preferably 2418 or less, and yet even more preferably 818 or less. When the number average molecular weight is not higher than the upper limit, the viscosity of the reaction liquid becomes more appropriate, and efficiency of dissolution of the polyamide resin (a) and the depolymerization reaction tend to further improve.

In the method for manufacturing a cyclic lactam of the present embodiment, the used amount of the polyphosphoric acid per 100 parts by mass of the polyamide resin (a) (added amount to the reaction system) is 1 part by mass or more, preferably 10 parts by mass or more, more preferably 40 parts by mass or more, even more preferably 50 parts by mass or more, yet even more preferably 70 parts by mass or more, yet even more preferably 90 parts by mass or more, and yet even more preferably 100 parts by mass or more. When the used amount is not lower than the lower limit, the depolymerization reaction is more effectively promoted due to high catalytic activity, and thus a cyclic lactam tends to be formed with higher selectivity. The used amount of the polyphosphoric acid per 100 parts by mass of the polyamide resin (a) (added amount to the reaction system) is preferably 10000 parts by mass or less, more preferably 1000 parts by mass or less, even more preferably 500 parts by mass or less, yet even more preferably 300 parts by mass or less, and yet even more preferably 200 parts by mass or less. When the used amount is not higher than the upper limit, reduction in raw material costs and reduction in energy during purification by distillation of the cyclic lactam can be attempted.
Note that, in a case where the polyamide resin (a) is continuously supplied to the reaction system to manufacture a cyclic lactam, the amount of the polyphosphoric acid present in the reaction system preferably satisfies the range described above.

In the present embodiment, the water content of the polyphosphoric acid (polyphosphoric acid itself, which does not include water) supplied to the reaction system is preferably 100 mass% or less, more preferably 50 mass% or less, even more preferably 10 mass% or less, yet even more preferably 1 mass% or less, yet even more preferably 0.1 mass% or less, and yet even more preferably 0.001 mass% or less, with respect to the mass of the polyphosphoric acid. When the water content is not higher than the upper limit, selective formation of a cyclic lactam and simple and easy purification by distillation of the cyclic lactam can be attempted. The lower limit of the water content of the polyphosphoric acid supplied to the reaction system may be 0 mass%; however, for example, even when the water content is 0.0001 mass% or more, required performance can be adequately satisfied.

### Solvent

In the method for manufacturing a cyclic lactam of the present embodiment, because the polyphosphoric acid functions as a solvent, a solvent (reaction solvent) may be used or no solvent may be used.

A first embodiment of the solvent in the method for manufacturing a cyclic lactam of the present embodiment is an embodiment where, when the depolymerization reaction of the polyamide resin (a) is performed, no solvent is used or an amount of a solvent is 10 mass% or less with respect to an amount of the polyphosphoric acid. In the first embodiment of the solvent, even when a configuration where a solvent is not substantially used is employed, the polyphosphoric acid serves a role of a solvent. In the first embodiment of the solvent, the amount of the solvent is preferably 5 mass% or less, more preferably 3 mass% or less, even more preferably 1 mass% or less, and yet even more preferably 0.1 mass% or less, with respect to the amount of the polyphosphoric acid.

In the first embodiment of the solvent described above, purification of a cyclic lactam is easily performed by reactive distillation or distillation after the reaction. Furthermore, energy costs tend to decrease.

In the first embodiment of the solvent described above, 95 mass% or more (preferably 99 mass% or more) of substances supplied to the reaction system (e.g., reaction tank) is preferably composed of the polyphosphoric acid and the polyamide resin (a). However, in the reaction, it is obvious that, in addition to a cyclic lactam which is a target product, formed water, an unreacted polyamide resin (a), a polyamide oligomer which is an intermediate product, and the like also exist in the reaction system.

The second embodiment of the solvent in the method for manufacturing a cyclic lactam of the present embodiment is an embodiment where the depolymerization reaction of the polyamide resin (a) is performed in the presence of a solvent having a high boiling point, and a boiling point of the solvent having the high boiling point is not lower than a temperature that is 10°C lower than a boiling point of a cyclic lactam to be manufactured. By using the solvent having the high boiling point, the viscosity of the reaction liquid decreases, the depolymerization reaction is promoted, and the yield of the distillation-purified product (cyclic lactam) tends to improve.

The boiling point of the solvent having the high boiling point is not lower than a temperature that is preferably 5°C lower, more preferably 3°C lower, even more preferably 1°C lower, yet even more preferably equal to or not lower than, yet even more preferably 1°C higher, and yet even more preferably 3°C higher, than the boiling point of the cyclic lactam to be manufactured. The upper limit of the boiling point of the solvent having the high boiling point is preferably not higher than a temperature that is 200°C higher than the boiling point of the cyclic lactam to be manufactured.

Furthermore, in a case where two or more types of the solvents having high boiling points are contained, the boiling point is taken as a boiling point of a solvent for which the content is the largest.

The viscosity at 25°C of the solvent having the high boiling point is preferably 1 cSt or more, and more preferably 10 cSt or more, and may be 20 cSt or more, and preferably 500 cSt or less, more preferably 300 cSt or less, and even more preferably 100 cSt or less. The viscosity of a solvent having a high boiling point is measured according to JIS Z 8803.

Examples of the solvent having the high boiling point include polysiloxanes and terminal-capped polyethylene glycols. Polyalkylsiloxanes (the alkyl group having from 1 to 3 carbons, and the average number of alkyl groups bonded to one Si atom is 1 or 2) are preferred, and a polydimethylsiloxane is more preferred.

Furthermore, the solvent having the high boiling point is preferably not an alcohol. By using no alcohol, decrease in catalytic activity due to alcoholysis of the polyphosphoric acid can be effectively prevented.

In the second embodiment of the solvent in the method for manufacturing a cyclic lactam of the present embodiment, the used amount of the solvent having the high boiling point is preferably 10 parts by mass or more, more preferably 50 parts by mass or more, and even more preferably 100 parts by mass or more, per 100 parts by mass of the polyphosphoric acid. When the used amount is not lower than the lower limit, the depolymerization reaction is promoted, and the yield of the distillation-purified product (cyclic lactam) tends to improve. Furthermore, the upper limit of the used amount of the solvent having the high boiling point is preferably 1000 parts by mass or less, more preferably 500 parts by mass or less, even more preferably 400 parts by mass or less, yet even more preferably 300 parts by mass or less, and yet even more preferably 200 parts by mass or less, per 100 parts by mass of the polyphosphoric acid. The used amount of not higher than the upper limit tends to make it possible to prevent the reaction liquid from being excessively diluted. More specifically, raw material costs can be reduced, and high depolymerization reactivity can be maintained.

In the method for manufacturing a cyclic lactam of the present embodiment, one type of the solvent having the high boiling point may be used, or two or more types of the solvents having the high boiling points may be used. When two or more types are used, the total amount is preferably within the range described above.

In the second embodiment of the solvent described above, 95 mass% or more (preferably 99 mass% or more) of substances supplied to the reaction system (e.g., reaction tank) is preferably composed of the polyphosphoric acid, the polyamide resin (a), and the solvent having the high boiling point. However, in the reaction, it is obvious that, in addition to a cyclic lactam which is a target product, formed water, an unreacted polyamide resin (a), a polyamide oligomer which is an intermediate product, and the like also exist in the reaction system.

A third embodiment of the solvent in the method for manufacturing a cyclic lactam of the present embodiment is an embodiment where, when the depolymerization reaction of the polyamide resin (a) is performed, no solvent other than the solvent having the high boiling point is used or an amount of a solvent other than the solvent having the high boiling point used is 10 mass% or less with respect to the amount of the polyphosphoric acid.

In the third embodiment of the solvent described above, 95 mass% or more (preferably 99 mass% or more) of substances supplied to the reaction system (e.g., reaction tank) is preferably composed of the polyphosphoric acid, the polyamide resin (a), and an optionally blended solvent having a high boiling point. However, in the reaction, it is obvious that, in addition to a cyclic lactam which is a target product, formed water, an unreacted polyamide resin (a), a polyamide oligomer which is an intermediate product, and the like also exist in the reaction system.

The details of the solvent having the high boiling point are synonymous with those of the solvent in the second embodiment. In a case where the solvent having the high boiling point is used, the type, and the preferred range of used amount, and the like of the solvent are the same as and/or similar to those of the solvent in the second embodiment.

### Depolymerization Reaction

Next, details of the depolymerization reaction will be described.

The pressure during the depolymerization reaction in the present embodiment is preferably 202.6 kPa or less, more preferably 150.0 kPa or less, even more preferably less than 101.3 kPa, yet even more preferably 13.3 kPa or less, and yet even more preferably 4.0 kPa or less. When the pressure is not higher than the upper limit, a cyclic lactam tends to be produced at a higher yield by reactive distillation. The lower limit of the pressure during the depolymerization reaction is preferably, for example, 0.01 kPa or more.

The pressure during the depolymerization reaction means a pressure that reached a stationary state after adjustment of the pressure in the reaction system by pressure reduction or pressurization. Note that, in the present embodiment, for example, the reaction may be performed in two or more stages of pressures, such as after reaction is performed under normal pressure for a certain time period, the reaction is performed under reduced pressure for a certain time period. In this case, the pressure at each stage preferably satisfies the range described above.

The temperature during the depolymerization reaction is preferably 170°C or higher, more preferably 190°C or higher, even more preferably 210°C or higher, and yet even more preferably 230°C or higher. When the temperature is not lower than the lower limit, the depolymerization reaction is promoted, and more efficient distillation of the cyclic lactam can be performed. Furthermore, the temperature during the depolymerization reaction is preferably 300°C or lower, more preferably 280°C or lower, and even more preferably 260°C or lower. When the temperature is not higher than the upper limit, energy required for the cyclic lactam production can be reduced, and generation of byproducts tends to be reduced.

The temperature during the depolymerization reaction means a temperature that reached a stationary state after the temperature is increased in the reaction system. Note that, in the present embodiment, the reaction may be performed in two or more stages of temperatures, such as after reaction is performed under a particular temperature for a certain time period, the reaction is performed in an increased temperature or decreased temperature for a certain time period. In this case, the reaction temperature at each stage preferably satisfies the range described above.

The depolymerization reaction may be performed without stirring but is preferably performed with stirring. By the stirring, the reaction can more effectively proceed. Furthermore, in the present embodiment, the stirring can be performed by using the polyphosphoric acid.

The stirring speed is properly adjusted based on the scale of the reaction (scale of the device) and the device structure and, for example, is preferably 10 rpm or more, more preferably 30 rpm or more, even more preferably 100 rpm or more, yet even more preferably 150 rpm or more, and yet even more preferably 200 rpm or more, and may be 300 rpm or more, or 500 rpm or more. When the stirring speed is not lower than the lower limit, the polyamide resin (a) and the polyphosphoric acid are homogenously mixed, and the depolymerization reaction tends to efficiently proceed. Furthermore, the stirring speed is preferably 1500 rpm or less, more preferably 1200 rpm or less, even more preferably 1000 rpm or less, yet even more preferably 800 rpm or less, and yet even more preferably 600 rpm or less, and may be 550 rpm or less. When the stirring speed is not higher than the upper limit described above, scattering of the reaction liquid to a side surface or a top surface of a reactor can be suppressed, and decrease in the yield can be effectively suppressed. Furthermore, clogging of the distillation flow pass can be effectively prevented.

The stirring speed means a temperature that reached a stationary state after adjustment of the stirring speed in the reaction system. Note that the stirring may be performed in two or more stages of stirring speeds, such as after stirring is performed at a particular speed for a certain time period, stirring may be performed further at another stirring speed. In this case, the stirring speed at each stage preferably satisfies the range described above.

Furthermore, as equipment used for stirring, equipment typically used for chemical products can be used. For example, the form of stirring blade is properly selected from a paddle form, an anchor form, a helical ribbon type, or the like.

In the present embodiment, when the depolymerization reaction is performed, no superheated steam may be used. By using no superheated steam, effective reduction in energy during purification and effective reduction in waste can be attempted.

Examples of the superheated steam include superheated steam of water, amines, and alcohols. More specific examples include superheated steam, methanol vapor, and ammonia vapor. The present embodiment is particularly useful from the viewpoint of being able to proceed the depolymerization reaction without use of superheated steam.

The depolymerization reaction in the present embodiment is typically not a hydrolysis reaction using water. Thus, in the present embodiment, at the time of depolymerization reaction, the depolymerization reaction is preferably performed while water is removed.

Furthermore, the depolymerization reaction in the present embodiment preferably proceeds by backbiting reaction, in which a terminal amino group or an amide bond in a main chain of the polyamide resin (a) attacks an amide bond in one unit next to itself.

### Distillation

In the method for manufacturing a cyclic lactam of the present embodiment, distillation is preferred. By performing distillation, a cyclic lactam that is a target product can be fractionated and purified. Furthermore, the reaction system may contain water due to a polyamide resin (a), which is a raw material, absorbing moisture or some of polyphosphoric acids being condensed during reaction; however, the water content can be removed by the distillation.

For the distillation, reactive distillation may be performed while the depolymerization reaction is performed, or distillation may be performed after the depolymerization reaction is performed. Needless to say, reactive distillation may be performed while the depolymerization reaction is performed, and distillation may be performed after the depolymerization reaction is performed.

The pressure at the time of performing distillation after the depolymerization reaction is performed is preferably less than 101.3 kPa, more preferably 13.3 kPa or less, even more preferably less than 4.0 kPa, yet even more preferably 2.7 kPa or less, and yet even more preferably 1.4 kPa or less. When the pressure is not higher than the upper limit, a cyclic lactam tends to be isolated at a higher yield. The lower limit of the pressure during the distillation is preferably, for example, 0.01 kPa or more.

### Supplying to Reaction System

In the method for manufacturing a cyclic lactam of the present embodiment, typically, manufacturing of a cyclic lactam is performed by supplying the polyamide resin (a) that is a raw material and the polyphosphoric acid to a reaction system.

The polyamide resin (a) may be supplied at once to the reaction system or may be continuously supplied to the reaction system. In a case where the polyamide resin (a) is continuously supplied to the reaction system, the amount of the polyphosphoric acid present in the reaction system is preferably 1 part by mass or more per 100 parts by mass of the polyamide resin (a).

In the method for manufacturing a cyclic lactam of the present embodiment, the polyphosphoric acid is supplied to the reaction system but, at this time, the polyphosphoric acid may be supplied at once to the reaction system or may be continuously supplied to the reaction system. From the viewpoint of industrial production, continuous supplying is preferred. Furthermore, reuse of the polyphosphoric acid is also preferred.

As manufacturing of the cyclic lactam proceeds, the polyphosphoric acid supplied to the reaction system may have a degree of polymerization higher than that of the polyphosphoric acid supplied to the reaction system. The method for manufacturing a cyclic lactam of the present embodiment may include regenerating a polyphosphoric acid (B3) by hydrolyzing a polyphosphoric acid (B2) having a degree of polymerization higher than that of the polyphosphoric acid supplied to the reaction system.

In the present embodiment, in particular, a polyphosphoric acid (B3) can be regenerated in the reaction system by hydrolyzing a polyphosphoric acid (B2) having a degree of polymerization higher than that of the polyphosphoric acid supplied to the reaction system.

Furthermore, as the polyphosphoric acid supplied to the reaction system, the regenerated polyphosphoric acid (B3) can be used.

Note that polyphosphoric acid (B2) having a degree of polymerization higher than that of the polyphosphoric acid supplied to the reaction system preferably contains, for example, a compound represented by Formula (P2) below.

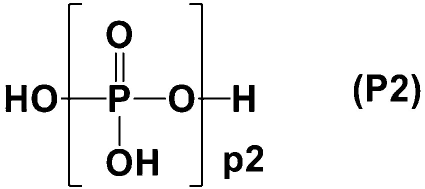

In Formula (P2), p2 is preferably from 3 to 130.

The polyphosphoric acid (B2) is typically a mixture of compounds represented by Formula (P2) having different numerical values of p2.

In a case where the polyphosphoric acid contains a compound represented by Formula (P1) and the polyphosphoric acid (B2) contains a compound represented by Formula (P2), p2 - p1, which is a difference between the average value of p2 of the compound represented by Formula (P2) contained in the polyphosphoric acid (B2) and the average value of p1 of the compound represented by Formula (P1) contained in the polyphosphoric acid supplied to the reaction system, is 1 or more, 2 or more, or 3 or more, and 30 or less, 20 or less, 10 or less, or 5 or less, for example.

### Embodiments

Preferred embodiments of the method for manufacturing a cyclic lactam will be described below. It is needless to say that the present invention is not limited to these embodiments.

The first embodiment of the method for manufacturing a cyclic lactam is a method for manufacturing a cyclic lactam where reactive distillation is performed while the depolymerization reaction is performed; the depolymerization reaction is performed at a temperature of 170°C to 300°C; when the depolymerization reaction is performed, superheated steam is not used; and when the depolymerization reaction is performed, no solvent is used or an amount of a solvent is 10 mass% or less with respect to an amount of the polyphosphoric acid.

By performing the first embodiment, the manufacturing time of the cyclic lactam can be shortened, and the cyclic lactam can be efficiently manufactured.

The second embodiment of the method for manufacturing a cyclic lactam is a method for manufacturing a cyclic lactam, where reactive distillation is performed while the depolymerization reaction is performed; the depolymerization reaction is performed at a temperature of 170°C to 300°C; when the depolymerization reaction is performed, superheated steam is not used; the depolymerization reaction is performed in the presence of a solvent having a high boiling point, and a boiling point of the solvent having the high boiling point is not lower than a temperature that is 10°C lower than a boiling point of a cyclic lactam to be manufactured; and the solvent having the high boiling point is not an alcohol.
According to the second embodiment, the viscosity of the reaction liquid can be reduced, and thus the depolymerization reaction can be promoted and the manufacturing time of the cyclic lactam can be shortened by reactive distillation.

The third embodiment of the method for manufacturing a cyclic lactam is a method for manufacturing a cyclic lactam where distillation is performed after the polymerization reaction is performed; the depolymerization reaction is performed at a temperature of 170°C to 300°C; when the depolymerization reaction is performed, superheated steam is not used; and when the depolymerization reaction is performed, no solvent is used or an amount of a solvent is 10 mass% or less with respect to an amount of the polyphosphoric acid.

According to the third embodiment, distillation purification can be performed at a temperature lower than that at the time of the depolymerization.

The fourth embodiment of the method for manufacturing a cyclic lactam is a method for manufacturing a cyclic lactam, where distillation is performed after the depolymerization reaction is performed; the depolymerization reaction is performed at a temperature of 170°C to 300°C; when the depolymerization reaction is performed, superheated steam is not used; the depolymerization reaction is performed in the presence of a solvent having a high boiling point, and a boiling point of the solvent having the high boiling point is not lower than a temperature that is 10°C lower than a boiling point of a cyclic lactam to be manufactured; and the solvent having the high boiling point is not an alcohol.

According to the fourth embodiment, the viscosity of the reaction liquid can be reduced, and thus the depolymerization reaction can be promoted and distillation purification can be performed at a temperature lower than that at the time of the depolymerization.

### Device for Manufacturing Cyclic Lactam

In the present embodiment, a device for manufacturing a cyclic lactam using the method for manufacturing the cyclic lactam of the present embodiment described above will be further described. FIG. 1 is a schematic view of an example of a device for manufacturing a cyclic lactam. In FIG. 1, 1 is a reactor, 2 is a container to hold a cyclic lactam, 3 is a flow pass, 4 is a pressure regulator, 5 is a heated part, 6 is an apparatus for removing water, and 7 is a container for collecting water. The device for manufacturing a cyclic lactam of the present embodiment will be described below with reference to FIG. 1. However, it is needless to say that the device for manufacturing a cyclic lactam of the present embodiment is not limited to FIG. 1.

Specifically, the present embodiment describes a device for manufacturing a cyclic lactam, which performs the method for manufacturing a cyclic lactam of the present embodiment described above and which has the following equipment:
(1) a reactor 1 configured to perform a depolymerization reaction by heating a mixture A containing a polyamide resin (a) and a polyphosphoric acid, to produce a cyclic lactam, and to form a mixture B containing the polyphosphoric acid and the cyclic lactam;
(2) a container 2 configured to hold the cyclic lactam vaporized from the mixture B in the reactor 1; and
(3) a flow pass 3 configured to connect the reactor 1 and the container 2 and to transfer the vaporized cyclic lactam from the reactor 1 to the container 2.

The mixture B typically contains a polyamide resin containing a repeating unit represented by Formula (a) and/or a polyamide oligomer containing a repeating unit represented by Formula (a).

In the mixture A containing the polyamide resin (a) and the polyphosphoric acid and the mixture B containing the polyphosphoric acid and the cyclic lactam, the polyphosphoric acid is synonymous with the polyamide resin (a) and the polyphosphoric acid that are supplied to the reaction system described above. For the ratio, preferred range, and the like, the description of "Polyamide Resin Containing Repeating Unit Represented by Formula (a) (Polyamide Resin (a))" and "Polyphosphoric Acid" described above can be considered. Furthermore, the mixture A and the mixture B may contain a solvent and, for the details of the solvent, the description of "Solvent" can be considered.

For the details of the depolymerization reaction, the description of "Depolymerization Reaction" described above can be considered.

Examples of the reactor 1 include those made of stainless steel to which glass lining or an anodic protection method is applied, or tantalum.

Examples of the container 2 configured to hold the cyclic lactam vaporized from the mixture B in the reactor 1 include a container made of at least one type selected from the group consisting of stainless steel to which glass lining is applied, stainless steel (SUS), tantalum-titanium, Hastelloy, Teflon (trade name) lining, nickel, zirconium, copper, and carbon net (CS).

Examples of the flow pass 3 configured to connect the reactor 1 and the container 2 and to transfer the vaporized cyclic lactam from the reactor 1 to the container 2 (indicated by a broken line in FIG. 1 for convenience) include a flow pass made of stainless steel and/or titanium.

Note that a valve, a joint, or the like may be provided in between the reactor 1 and the flow pass 3 and/or in between the container 2 and the flow pass 3. By providing a valve, the pressure or flow rate can be adjusted. Furthermore, the pressure or flow rate may be adjusted by providing a valve in the middle of the flow pass 3.

Furthermore, the device for manufacturing a cyclic lactam of the present embodiment preferably includes a reactor 1, a container 2, and a pressure regulator 4 configured to maintain a pressure of 202.6 kPa or less in the flow pass 3. Examples of the pressure regulator 4 include a reciprocating piston pump and a vacuum pump such as a rotative pump. In the device for manufacturing a cyclic lactam of the present embodiment, typically, the entire region where the raw material or the product may pass through in the device including the reactor 1, the container 2, and the flow pass 3 may be regulated by one pressure regulator 4 set up in any one of the reactor 1, the container 2, or the flow pass 3 or may be regulated by pressure regulators 4 set up in each of the reactor 1, the container 2, and the flow pass 3.

The pressure regulator 4 is only required to be connected to a part freely selected from the reactor 1, the container 2, and/or the flow pass 3 described above, and is preferably connected to the flow pass 3. With such a configuration, a compound having a boiling point lower than a cyclic lactam mixed with (a little amount of) water or the like can be efficiently isolated. Note that a valve, a joint, or the like may be provided in between the flow pass 3 and the pressure regulator 4.

Furthermore, the pressure regulator 4 is preferably connected in between the flow pass 3 and the apparatus for removing water 6 described below.

Furthermore, in the present embodiment, a heated part 5 configured to maintain the temperature in the flow pass 3 to not lower than the melting point (preferably not lower than a temperature 1°C higher than the melting point, or not higher than a temperature 50°C higher than the melting point) of the cyclic lactam is preferably included. By providing the heated part 5, clogging due to cyclic lactam deposition can be effectively suppressed. Examples of the heated part 5 include heating medium such as warm water and steam at 50°C or higher or the like, and a heat exchanger that circulates the heating medium.

Furthermore, in the present embodiment, an apparatus 6 for removing water from the reactor 1 is preferably included. By providing the apparatus 6 for removing water, hydrolysis of the cyclic lactam and the polyphosphoric acid can be effectively suppressed. Examples of the apparatus 6 for removing water include a condenser and a molecular sieve column. Furthermore, the device for manufacturing a cyclic lactam of the present embodiment preferably includes a container 7 for collecting removed water.

The cyclic lactam manufactured by the method or the device for manufacturing a cyclic lactam of the present embodiment can be used as a polyamide resin, can be reused as a raw material for fibers, or can be used as a raw material for a medical polymer.

### Examples

Hereinafter, the present invention will be described more specifically with reference to Examples. Materials, amounts used, proportions, processing details, and processing procedures described in the following Examples can be appropriately changed without departing from the spirit of the present invention. Thus, the scope of the present invention is not limited to the specific examples described below.

If it is difficult to obtain the measuring instrument or the like used in Examples due to discontinuation or the like, another instrument with equivalent performance can be used for the measurement.

### Raw Material

Polyamide 6 (nylon 6): manufacturer: Sigma Aldrich; product number: 181110; viscosity average molecular weight:10000
Polyphosphoric acid: manufacturer: FUJIFILM Wako Pure Chemical Corporation; product number: 166-03035
Concentrated hydrochloric acid: manufacturer: FUJIFILM Wako Pure Chemical Corporation; product number: 080-01066; water content: 63.8 mass%
Concentrated sulfuric acid: manufacturer: FUJIFILM Wako Pure Chemical Corporation; product number: 192-04696
Phosphoric acid: H₃PO₄; manufacturer: FUJIFILM Wako Pure Chemical Corporation; product number: 167-02166; water content: 14.4 mass%
Polydimethylsiloxane (viscosity: 10 cSt (25°C)): manufacturer: Sigma Aldrich; product number: 378321; boiling point: higher than 140°C/0.002 mmHg (when a boiling point is calculated by a boiling point conversion formula by Hildebrand, the boiling point is 460°C at 101.3 kPa)
Polydimethylsiloxane (viscosity: 50 cSt (25°C)): manufacturer: Sigma Aldrich; product number: 378356; boiling point: higher than 140°C/0.002 mmHg (when a boiling point is calculated by a boiling point conversion formula by Hildebrand, the boiling point is 460°C at 101.3 kPa)

### Example 1

In an eggplant flask equipped with a condenser provided with a drying tube and equipped with a stirring apparatus, 91 parts by mass of polyphosphoric acid was blended with respect to 1 part by mass of polyamide 6, and then the mixture was reacted at 220°C in a pressure of 101.3 kPa for 6 hours using an oil bath while the mixture was stirred at a speed of 600 rpm.

The reaction liquid after the reaction was dissolved in heavy water and analyzed by the proton nuclear magnetic resonance method (¹H-NMR), and ε-caprolactam (ε-CL) was produced with high selectivity of 92 mol%.

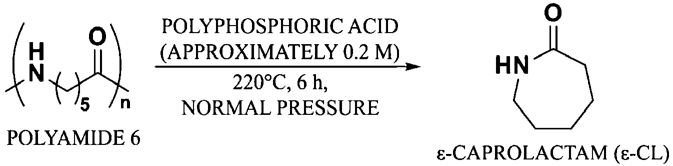

### Comparative Example 1

The same procedure was performed as in Example 1 except for blending 74 parts by mass of phosphoric acid with respect to 1 part by mass of the polyamide 6 and reacting the mixture at 220°C for 6 hours while stirring.

The products were 6-aminohexanoic acid (AHA) and a water-soluble polyamide oligomer.

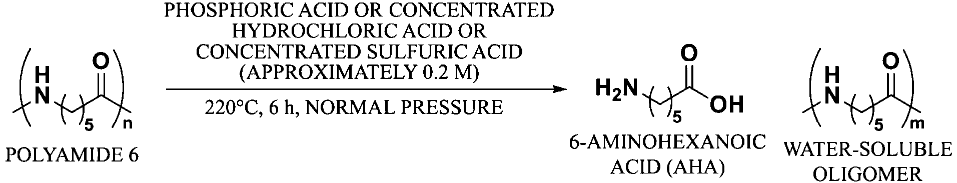

### Comparative Example 2

The same procedure was performed as in Example 1 except for blending 52 parts by mass of concentrated hydrochloric acid with respect to 1 part by mass of the polyamide 6 and reacting the mixture at 220°C for 6 hours while stirring.

### Comparative Example 3

The same procedure was performed as in Example 1 except for blending 81 parts by mass of concentrated sulfuric acid with respect to 1 part by mass of the polyamide 6 and reacting the mixture at 220°C for 6 hours while stirring.

The products were 6-aminohexanoic acid (AHA) and a water-soluble polyamide oligomer.

### Comparative Example 4

With respect to 1 part by mass of the polyamide 6, 0.005 parts by mass of polyphosphoric acid was blended and the mixture was reacted at 220°C for 6 hours. During the reaction, the most of the mixture remained as a solid, and stirring was difficult.

After the reaction, 99 mass% (0.99 parts by mass) or more of the raw material polyamide 6 that was insoluble in water or ethanol was recovered.

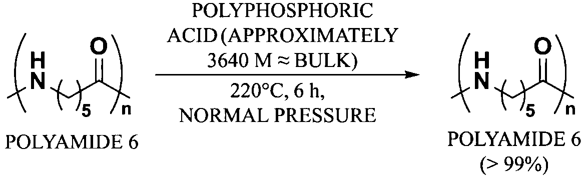

The results of Example 1 and Comparative Examples 1 to 4 are shown in Table 1 below.

**[Table 1]**

| | Acid catalyst | | Product after reaction (%)^{a} |
|---|---|---|---|
| | Compound name | Part(s) by mass | |
| Example 1 | Polyphosphoric acid | 91 | ε-CL (92), AHA + oligomer (8) |
| Comparative Example 1 | Phosphoric acid | 74 | AHA + oligomer (>99) |
| Comparative Example 2 | Concentrated hydrochloric acid | 52 | AHA + oligomer (>99) |
| Comparative Example 3 | Concentrated sulfuric acid | 81 | AHA + oligomer (>99) |
| Comparative Example 4 | Polyphosphoric acid | 0.005 | Polyamide 6 (>99) |

| | | | |
|---|---|---|---|
| ^{a 1}H-NMR analysis | | | |

The results described above indicate that the method of the present invention was able to manufacture the cyclic lactam with high selectivity. On the other hand, cyclic lactam was not produced in cases where an acid other than the polyphosphoric acid was used (Comparative Examples 1 to 3). Furthermore, almost no cyclic lactam was produced for a case where the content of the polyphosphoric acid was small even though the polyphosphoric acid was used (Comparative Example 4).

### Example 2

Distillation apparatus including an eggplant flask equipped with a stirring apparatus (reactor), an eggplant flask for recovering a distillation-purified product (container configured to hold the cyclic lactam), and a Liebig condenser with a T-shaped tube (flow pass) was prepared. In the eggplant flask that was a reactor, 1 part by mass of polyamide 6 and 1 part by mass of polyphosphoric acid were blended. Then, reactive distillation was performed by reducing the pressure to 20 mmHg (2.6645 kPa) by using a diaphragm vacuum pump with a pressure controller (pressure regulator) while the mixture was stirred at a speed of 300 rpm at 230°C for 6 hours using an oil bath. The water generated in a very small amount was collected by using a cooling trap (apparatus for removing water) placed in between the distillation apparatus and the diaphragm vacuum pump.

¹H-NMR analysis revealed that the distillation-purified product was ε-CL (yield: 56%). T¹H-NMR analysis revealed that the residue was a mixture of ε-CL (13%) and oligomers (31%).

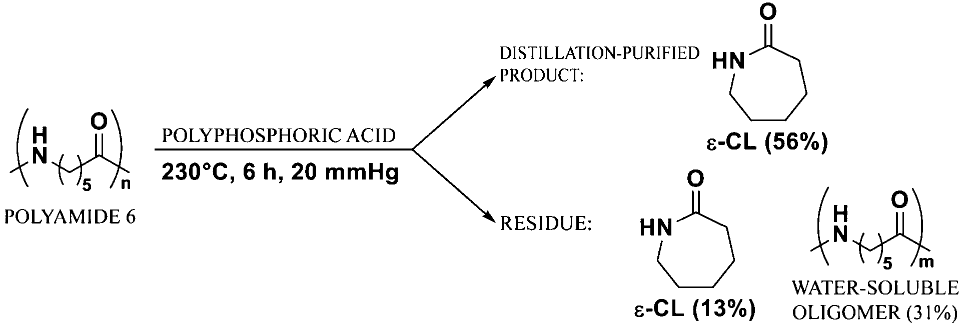

### Example 3

The reactive distillation was performed by the same method as in Example 2 except for reducing the pressure to 10 mmHg (1.3332 kPa) using a diaphragm vacuum pump.

¹H-NMR analysis revealed that he distillation-purified product was ε-CL (yield: 66%). ¹H-NMR analysis revealed that the residue was a mixture of ε-CL (2%) and oligomers (32%).

### Example 4

The reactive distillation was performed by the same method as in Example 2 except for reducing the pressure to 3 mmHg (0.39997 kPa) using a diaphragm vacuum pump.

¹H-NMR analysis revealed that the distillation-purified product was ε-CL (yield: 71%). ¹H-NMR analysis revealed that the residue was a mixture of ε-CL (1%) and oligomers (28%).

### Comparative Example 5

The same procedure was performed as in Example 2 except for changing the polyphosphoric acid to the same amount of phosphoric acid.

The distillation-purified product was ε-caprolactam (ε-CL) but the yield was 10%, which was low.

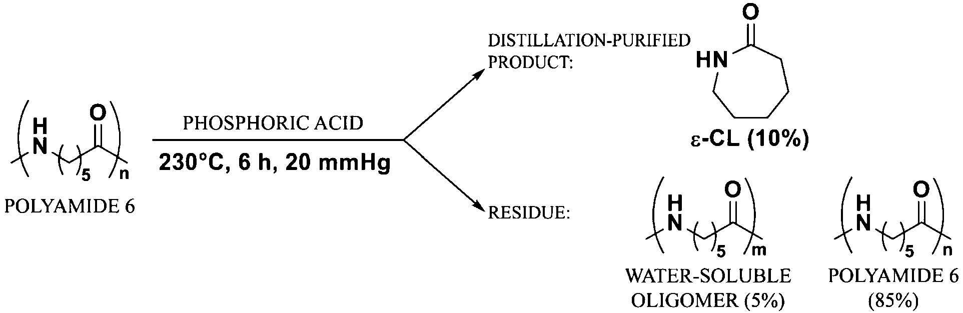

The results of Examples 2 to 4 and Comparative Example 5 are shown in Table 2 below.

**[Table 2]**

| | Catalyst | | Pressure | Product after reaction | |
|---|---|---|---|---|---|
| | Compound name | Part(s) by mass | Degree of vacuum (mmHg) | Distillation-purified product (%)^{a} | Residue after distillation (%)^{b} |
| Example 2 | Polyphosph oric acid | 1 | 20 | ε-CL (56) | ε-CL (13), oligomer (31) |
| Example 3 | Polyphosph oric acid | 1 | 10 | ε-CL (66) | ε-CL (2), oligomer (32) |
| Example 4 | Polyphosph oric acid | 1 | 3 | ε-CL (71) | ε-CL (1), oligomer (28) |
| Comparative Example 5 | Phosphoric acid | 1 | 20 | ε-CL (10) | Oligomer (5), polyamide 6 (85) |

| | | | | | |
|---|---|---|---|---|---|
| ^{a}Isolated yield, ^{b 1}H-NMR analysis | | | | | |

The results described above indicate that the method of the present invention was able to manufacture the cyclic lactam with high selectivity (Examples 2 to 4). On the other hand, cyclic lactam was manufactured but the yield thereof was low in a case where an acid other than the polyphosphoric acid was used (Comparative Example 5).

### Example 5

The reactive distillation was performed by blending 1 part by mass of polyphosphoric acid and 1 part by mass of polydimethylsiloxane (viscosity: 10 cSt) with respect to 1 part by mass of polyamide 6 and stirring at 300 rpm at 230°C and at a pressure of 20 mmHg (2.6645 kPa) for 6 hours.

¹H-NMR analysis revealed that the distillation-purified product was ε-CL (yield: 89%). ¹H-NMR analysis revealed that the residue was a mixture of ε-CL (4%) and oligomers (8%).

### Example 6

The reactive distillation was performed by blending 1 part by mass of polyphosphoric acid and 1 part by mass of polydimethylsiloxane (viscosity: 50 cSt) with respect to 1 part by mass of polyamide 6 and stirring at 300 rpm at 230°C and at a pressure of 20 mmHg (2.6645 kPa) for 6 hours.

¹H-NMR analysis revealed that the distillation-purified product was ε-CL (yield: 78%). ¹H-NMR analysis revealed that the residue was a mixture of ε-CL (8%) and oligomers (14%).

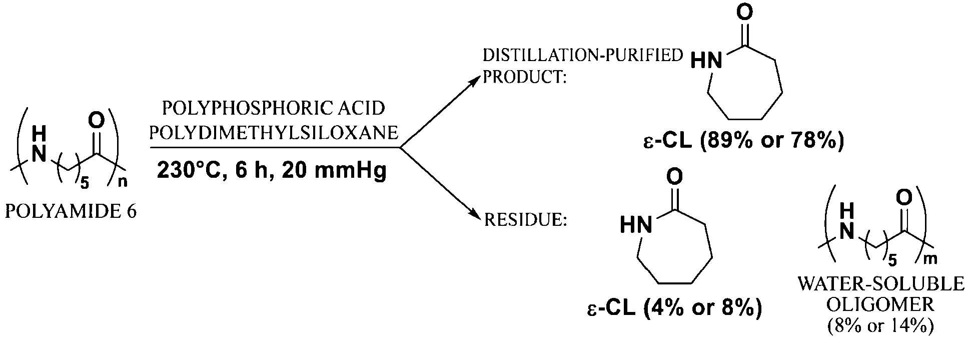

**[Table 3]**

| | Polydimethylsiloxane | | Product after reaction | |
|---|---|---|---|---|
| | Viscosity | Part(s) by mass | Distillation-purified product (%)^{a} | Residue after distillation (%)^{b} |
| Example 2 | - | - | ε-CL (56) | ε-CL (13), oligomer (31) |
| Example 5 | 10 cSt | 1 | ε-CL (89) | ε-CL (4), oligomer (8) |
| Example 6 | 50 cSt | 1 | ε-CL (78) | ε-CL (8), oligomer (14) |

| | | | | |
|---|---|---|---|---|
| ^{a}Isolated yield, ^{b 1}H-NMR analysis | | | | |

The results described above indicate that the depolymerization reaction is promoted due to reduction in the viscosity of the reaction liquid caused by addition of a solvent having a high boiling point, such as polydimethylsiloxane, the interaction between the polyphosphoric acid and the cyclic lactam becomes weak due to the polyphosphoric acid being diluted, and thus the yield of the distillation-purified product (cyclic lactam) improves.

### Example 7

Depolymerization of the polyamide 6 was attempted by using the polyphosphoric acid regenerated by hydrolysis.

Specifically, by using the eggplant flask after the operation of Example 2 was performed, pure water was added to the residue thereof in a manner that the amount of the pure water was 5 wt% with respect to the amount of the polyphosphoric acid. Thereafter, by using an oil bath, heating was performed at 75°C for 30 minutes. Thereafter, to a total of 1 part by mass of polyamide 6 prepared by adding the polyamide 6 in an amount that is the same as the amount (produced amount of caprolactam) of the polyamide 6 consumed in the operation of Example 2, 1 part by mass of polyphosphoric acid was blended. Then, reactive distillation was performed by reducing the pressure to 20 mmHg (2.6645 kPa) by using a diaphragm vacuum pump with a pressure controller (pressure regulator) while the mixture was stirred at a speed of 300 rpm at 230°C for 6 hours using an oil bath.

The water generated in a very small amount was collected by using a cooling trap (apparatus for removing water) placed in between the distillation apparatus and the diaphragm vacuum pump.

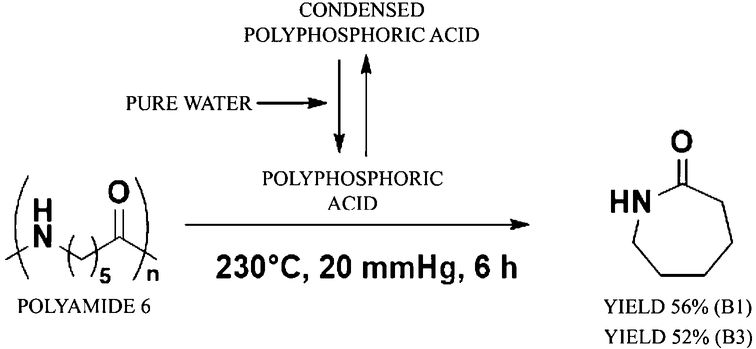

**[Table 4]**

| | Catalyst | | Amount of water added | Product after reaction |
|---|---|---|---|---|
| | Compound name | Part(s) by mass | wt% | Distillation-purified product (%)^{a} |
| Example 2 | Polyphosphoric acid (B1) | 1 | - | ε-CL (56) |
| Example 7 | Polyphosphoric acid (B3) | 1 | 5 | ε-CL (52) |

| | | | | |
|---|---|---|---|---|
| ^{a}Isolated yield | | | | |

In Table 4 above, (B1) is the polyphosphoric acid supplied to the reaction system, and (B3) is the regenerated polyphosphoric acid.

### Reference Signs List

1 Reactor
2 Container to hold cyclic lactam
3 Flow pass
4 Pressure regulator
5 Heated part
6 Apparatus for removing water
7 Container for collecting water

## Claims

1. A method for manufacturing a cyclic lactam, the method comprising performing a depolymerization reaction by using 1 part by mass or more of a polyphosphoric acid per 100 parts by mass of a polyamide resin containing a repeating unit represented by Formula (a): 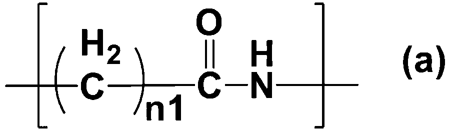 where n1 is an integer of 3 to 22, and n1 may be a different value for each of the repeating unit.

2. The method for manufacturing a cyclic lactam according to claim 1, wherein 40 parts by mass or more of the polyphosphoric acid is used per 100 parts by mass of the polyamide resin.

3. The method for manufacturing a cyclic lactam according to claim 1, wherein from 50 to 10000 parts by mass of the polyphosphoric acid is used per 100 parts by mass of the polyamide resin.

4. The method for manufacturing a cyclic lactam according to claim 1, wherein the depolymerization reaction is performed under a pressure of 202.6 kPa or less.

5. The method for manufacturing a cyclic lactam according to claim 1, wherein reactive distillation is performed while the depolymerization reaction is performed.

6. The method for manufacturing a cyclic lactam according to claim 1, wherein distillation is performed after the depolymerization reaction is performed.

7. The method for manufacturing a cyclic lactam according to claim 1, wherein the depolymerization reaction is performed at a temperature of 170°C to 300°C.

8. The method for manufacturing a cyclic lactam according to claim 1, wherein superheated steam is not used when the depolymerization reaction is performed.

9. The method for manufacturing a cyclic lactam according to claim 1, wherein, when the depolymerization reaction is performed, no solvent is used or an amount of a solvent is 10 mass% or less with respect to an amount of the polyphosphoric acid.

10. The method for manufacturing a cyclic lactam according to claim 1, wherein the depolymerization reaction is performed in the presence of a solvent having a high boiling point, and a boiling point of the solvent having the high boiling point is not lower than a temperature that is 10°C lower than a boiling point of a cyclic lactam to be manufactured.

11. The method for manufacturing a cyclic lactam according to claim 10, wherein the solvent having the high boiling point is not an alcohol.

12. The method for manufacturing a cyclic lactam according to claim 1, wherein
reactive distillation is performed while the depolymerization reaction is performed;
the depolymerization reaction is performed at a temperature of 170°C to 300°C;
when the depolymerization reaction is performed, superheated steam is not used; and
when the depolymerization reaction is performed, no solvent is used or an amount of a solvent is 10 mass% or less with respect to an amount of the polyphosphoric acid.

13. The method for manufacturing a cyclic lactam according to claim 1, wherein
reactive distillation is performed while the depolymerization reaction is performed;
the depolymerization reaction is performed at a temperature of 170°C to 300°C;
when the depolymerization reaction is performed, superheated steam is not used;
the depolymerization reaction is performed in the presence of a solvent having a high boiling point, and a boiling point of the solvent having the high boiling point is not lower than a temperature that is 10°C lower than a boiling point of a cyclic lactam to be manufactured; and
the solvent having the high boiling point is not an alcohol.

14. The method for manufacturing a cyclic lactam according to claim 1, wherein,
after the depolymerization reaction is performed, distillation is performed;
the depolymerization reaction is performed at a temperature of 170°C to 300°C;
when the depolymerization reaction is performed, superheated steam is not used; and
when the depolymerization reaction is performed, no solvent is used or an amount of a solvent is 10 mass% or less with respect to an amount of the polyphosphoric acid.

15. The method for manufacturing a cyclic lactam according to claim 1, wherein,
after the depolymerization reaction is performed, distillation is performed;
the depolymerization reaction is performed at a temperature of 170°C to 300°C;
when the depolymerization reaction is performed, superheated steam is not used;
the depolymerization reaction is performed in the presence of a solvent having a high boiling point, and a boiling point of the solvent having the high boiling point is not lower than a temperature that is 10°C lower than a boiling point of a cyclic lactam to be manufactured; and
the solvent having the high boiling point is not an alcohol.

16. The method for manufacturing a cyclic lactam according to claim 1, wherein a molecular weight of the polyphosphoric acid is 177 or more and 10000 or less.

17. The method for manufacturing a cyclic lactam according to claim 1, wherein the polyamide resin is at least one type selected from the group consisting of nylon 4, nylon 5, nylon 6, nylon 11, and nylon 12.

18. The method for manufacturing a cyclic lactam according to claim 1, wherein a cyclic lactam is manufactured by continuously supplying the polyamide resin to a reaction system.

19. The method for manufacturing a cyclic lactam according to claim 1, wherein the polyphosphoric acid is directly supplied to a reaction system.

20. The method for manufacturing a cyclic lactam according to claim 19, wherein a water content of the polyphosphoric acid supplied to the reaction system is 100 mass% or less with respect to a mass of the polyphosphoric acid.

21. The method for manufacturing a cyclic lactam according to claim 19, wherein a polyphosphoric acid (B2) having a degree of polymerization higher than a degree of polymerization of the polyphosphoric acid supplied to the reaction system is hydrolyzed and a polyphosphoric acid (B3) is regenerated.

22. The method for manufacturing a cyclic lactam according to claim 19, wherein a polyphosphoric acid (B2) having a degree of polymerization higher than a degree of polymerization of the polyphosphoric acid supplied to the reaction system is hydrolyzed and a polyphosphoric acid (B3) is regenerated in the reaction system.

23. The method for manufacturing a cyclic lactam according to claim 21, wherein the polyphosphoric acid (B3) is used as a polyphosphoric acid to be supplied to the reaction system.

24. A device for manufacturing the cyclic lactam configured to perform the method for manufacturing a cyclic lactam described in any one of claims 1 to 23, the device comprising:
(1) a reactor configured to perform a depolymerization reaction by heating a mixture A containing a polyamide resin containing a repeating unit represented by Formula (a) and a polyphosphoric acid, to produce a cyclic lactam, and to form a mixture B containing the polyphosphoric acid and the cyclic lactam;
(2) a container configured to hold the cyclic lactam vaporized from the mixture B in the reactor; and
(3) a flow pass configured to connect the reactor and the container and to transfer the vaporized cyclic lactam from the reactor to the container.

25. The device for manufacturing a cyclic lactam according to claim 24, further comprising a pressure regulator configured to maintain a pressure of 202.6 kPa or less in the reactor, the container, and the flow pass.

26. The device for manufacturing a cyclic lactam according to claim 25, wherein the pressure regulator is connected to at least one selected from the group consisting of the reactor, the container, and the flow pass.

27. The device for manufacturing a cyclic lactam according to claim 24, further comprising a heated part configured to maintain a temperature in the flow pass to not lower than a melting point of the cyclic lactam.

28. The device for manufacturing a cyclic lactam according to claim 24, further comprising an apparatus for removing water from the reactor.
